# EUROPEAN PATENT SPECIFICATION

(11) **EP 2 742 326 B1**
(45) Date of publication and mention of the grant of the patent: **26.08.2015**
(21) Application number: 11832152.0
(22) Date of filing: 20.09.2011
(51) Int. Cl.: G01F 1/74, G01N 23/12

(54) **AN APPARATUS FOR MEASURING THE COMPOSITION OF A MULTI-PHASE MIXTURE FLOW**
VORRICHTUNG ZUM MESSEN DER ZUSAMMENSETZUNG EINER MEHRPHASENMISCHUNGSSTRÖMUNG
APPAREIL DE MESURE DE LA COMPOSITION D'UN FLUX DE MÉLANGE MULTIPHASÉ

(43) Date of publication of application: 18.06.2014
(73) Proprietor: Siemens Aktiengesellschaft, 80333 München (DE)
(72) Inventor: POLIKHOV, Stepan Alexandrovich, Ramenskoe 140105 (RU); SCHULZ, Reiner Franz, Erlangen, 91054 (DE)
(86) International application number: PCT/RU2011/000716
(87) International publication number: WO 2013/043074

(56) References cited:
- WO-A1-97/29356
- WO-A1-2009/093927
- WO-A1-2011/005133
- US-A- 4 924 099
- US-A- 6 097 786

## Description

The invention relates to an apparatus and a method for measuring the composition of a multi-phase mixture flow.

In many industries, there is a need for techniques for measuring the composition of a multi-phase mixture flow. Particularly in the field of gas and oil industry, non-intrusive methods are known for measuring the multi-phase mixture flow emanating from an oil or gas well.

In document US 6,097,786, a method and apparatus for determining the composition of a multi-phase mixture flow based on X-ray radiation is disclosed. According to this measurement principle, the multi-phase mixture flow is irradiated with high energy and low energy X-ray radiation. The radiation which passes through the flow is measured by a multi-layer detector. As the attenuation of the radiation depends on the composition of the multi-phase mixture flow, the fractions of the different phases can be determined.

Document WO 97/29356 also describes a related apparatus for measuring the composition of a multi-phase mixture flow.

Document WO 2011/005133 A1 describes an apparatus and method for measuring the flow velocity of a multi-phase fluid mixture. To do so, images of the spatial distributions of the photons from X-ray sources are detected by detection means at different intervals of time. Based on those distributions, the flow rate of the fluid mixture can be determined.

In the prior art solutions for analysing a multi-phase mixture flow, the measurement apparatus needs to be calibrated manually. Particularly, the different absorption coefficients of the phases in the flow have to be determined in advance so that the composition of the flow can be calculated based on those absorption coefficients. Manual calibration is time-consuming because a sample of the flow has to be taken by a person at the location of the apparatus. Furthermore, due to the effort of manual calibration, this calibration is not repeated very often, leading to inaccurate results for the measured composition of the multi-phase mixture flow.

It is an object of the invention to provide an apparatus and a method for measuring the composition of a multi-phase mixture flow having the ability for self-calibration.

This object is solved by the apparatus according to claim 1 and the method according to claim 18. Preferred embodiments are described in the dependent claims.

The apparatus according to the invention measures the composition of a multi-phase mixture flow comprising at least one liquid phase, e.g. different liquids like oil and water, and at least one a gaseous phase, e.g. different kinds of gases. Particularly, the apparatus is for measuring a flow of liquid and gaseous hydrocarbons emanating from a well. The apparatus comprises a measurement tube forming a conduit for a flow of multi-phase mixture. The term "measurement tube" is to be interpreted broadly and can refer to any measurement section having an arbitrary cross-section, e.g. an rectangular or a circular cross-section. The apparatus further comprises radiation means for irradiating the multi-phase mixture in the measurement tube with electromagnetic radiation. A detection means is provided for detecting the radiation of the radiation means which passes through the multi-phase mixture in the measurement tube. Analysis means are used for determining the composition of the multi-phase mixture based on the detected radiation and calibration data of the at least one liquid phase and the at least one gaseous phase. The measurement principle used in the invention may be based on prior art methods. Particularly, the measurement may be based on the method disclosed in document US 6,097,786 where radiation at different energy levels is detected in order to determine the composition of a multi-phase mixture flow.

Contrary to prior art devices, a self-calibration unit is implemented in the apparatus of the invention. The unit comprises a calibration vessel which is arranged adjacent to the measurement tube such that in the operation of the apparatus the radiation means can irradiate the calibration vessel and the detection means can detect radiation of the radiation means passing through the calibration vessel.

The calibration vessel is connectable, i.e. is formed to be connected and/or is connected to the measurement tube such that the calibration vessel is filled with the multi-phase mixture or respective phases of the multi-phase mixture from the measurement tube. The calibration unit further comprises data acquisition means for acquiring calibration data from radiation detected by the detection means which passes through the calibration vessel when the calibration vessel is filled with the multi-phase mixture or respective phases of the multi-phase mixture from the measurement tube.

The invention has the advantage that an easy automatic calibration can be performed by filling a calibration vessel with multi-phase mixture and automatically acquiring calibration data via data acquisition means. Hence, there is no need for a manual calibration. The automatic calibration can be performed at regular intervals, ensuring a high accuracy of the measured composition of a multi-phase mixture flow.

In a preferred embodiment, the radiation of the radiation means is a high energy electromagnetic radiation with a photon energy of at least 10 KeV. Particularly, X-ray radiation and/or gamma radiation is well suited as this radiation is only partially absorbed by the multi-phase mixture, so that radiation can be detected by the detection means.

As mentioned above, prior art methods may be used for determining the composition of the multi-phase mixture flow, e.g. the method described in document US 6,097,786. To do so, the radiation means can generate at least two different radiation pulses, one pulse having a low energy level and the other pulse having a high energy level. Analogously, the detection means can detect the different radiation pulses. Furthermore, the analysis means is designed for determining the composition of the multi-phase mixture flow based on the detected different radiation pulses and the calibration data, where the calibration data,is acquired by the data acquisition means from the different radiation pulses detected by the detection means which pass through the calibration vessel when the calibration vessel is filled with the multi-phase mixture or respective phases of the multi-phase mixture from the measurement tube.

In a preferred embodiment, the calibration data comprises absorption coefficients for the phases in the multi-phase mixture with respect to the radiation of the radiation means. Those coefficients enable a calculation of the different fractions of the phases of the multi-phase mixture flow in the measurement tube.

In order to enable an easy calibration, the measurement conditions for the measurement tube and for the calibration vessel shall be similar. Hence, in a preferred embodiment, the measurement tube and the calibration vessel are made of the same material and/or have the same cross-section. Particularly, the measurement tube and/or the calibration vessel are made of beryllium bronze and/or carbon fiber and/or glassy carbon which are transmissive for electromagnetic radiation and particularly high energy electromagnetic radiation.

Furthermore, the measurement tube and/or the calibration vessel preferably have an elliptic cross-section or a cross-section in the form of an elongated hole. Those cross-sections at the one hand provide high resistance with respect to the pressure in the tube and the vessel and on the other hand ensure that the paths of the radiation beams through the tube and the vessel do not vary too much.

In a particularly preferred embodiment of the invention, the measurement tube and the calibration vessel are arranged symmetrically with respect to the radiation means and the detection means such that radiation reaching the detection means which has respectively passed through the measurement tube and the calibration vessel does not change when the positions of the measurement tube and the calibration vessel are interchanged. Hence, the measurement conditions for the tube and the vessel are substantially the same so that a straightforward calculation based on the calibration data without any conversions can be performed in order to determine the composition of a multi-phase mixture flow.

In order to provide similar conditions during calibration and measurement, the temperatures in the calibration vessel and the measurement tube should be the same. Hence, a section of the measurement tube and a section of the calibration vessel are preferably in direct contact with each other ensuring a good thermal transfer. Furthermore, the measurement tube and the calibration vessel are preferably surrounded by a thermal insulation such that the thermal conditions of the tube and the vessel are not affected by the environment.

The detection means in the apparatus of the invention can be implemented in various ways. Particular, the detection means may comprise one or more detection sensors. In a preferred embodiment, the detection means comprises a matrix detection means enabling a spatial resolution of the detected radiation and/or the detection means comprises two detection sensors, where one detection sensor is for detecting radiation passing through the measurement tube and the other detection sensor is for detecting radiation passing through the calibration vessel. By using a matrix detection means, different phases in the multi-phase mixture in the calibration vessel can be distinguished after a segregation step.

In another embodiment of the invention, the apparatus is designed such that during operation of the apparatus the measurement tube and the calibration vessel extend in the vertical direction enabling a gravitational stratification of the different phases in the multi-phase mixture inside the calibration vessel.

In a preferred variant of the invention, in order to connect the calibration vessel with a measurement tube, a valve system is provided comprising one or more valves and one or more conduits. The valve system is arranged between the measurement tube and the calibration vessel and is controllable by the data acquisition means. This system enables the calibration vessel to be filled with the multi-phase mixture or respective phases of the multi-phase mixture from the measurement tube.

In a preferred embodiment, the valve system comprises a sampling probe positioned in the measurement tube and connected via a first conduit including a first valve with the calibration vessel, where the calibration vessel is filled with the multi-phase mixture from the measurement tube when the first valve is opened.

Furthermore, the valve system preferably comprises a second conduit including a second valve, where the at least one gaseous phase of multi-phase mixture can be interchanged between the measurement tube and the calibration vessel when the second valve is opened.

The valve system may also comprise a third conduit including a third valve, where multi-phase mixture in the calibration vessel is fed back to the measurement tube when the third valve is opened, where the third conduit is preferably connected to a flow restriction in the measurement tube. This flow restriction enables a reduction of pressure in the measurement tube, thus enabling a good return flow to the measurement tube.

In another embodiment, the valve system comprises a fourth conduit including a fourth valve, where the at least one gaseous phase of multi-phase mixture in the calibration vessel is released from the calibration vessel when the fourth valve is opened. This embodiment may be used in order to measure the composition of stable and unstable gas condensates included in a gas condensate flow.

Besides the above described measurement of the composition of a multi-phase mixture, the apparatus of the invention may also enable the measurement of the flow rate based on images taken by the detection means. Preferably, the method as disclosed in document WO 2011/005133 A1 may be used in order to determine the flow rate. The whole disclosure of this document is incorporated by reference in this application.

Besides the above apparatus, the invention also refers to a method for calibrating this apparatus, comprising the steps of:
i) filling the calibration vessel with the multi-phase mixture of respective phases of the multi-phase mixture from the measurement tube;
ii) irradiating the calibration vessel with electromagnetic radiation from the radiation means and detecting the radiation which passes through the calibration vessel by the detection means;
iii) acquiring calibration data by the data acquisition means from the radiation detected by the detection means.

In a preferred embodiment of this method, the calibration vessel is subsequently filled with the at least one gaseous phase and the at least one liquid phase of the multi-phase mixture from the measurement tube or vice versa, where steps ii) and iii) are performed for both the at least one liquid phase and the at least one gaseous phase. This method is preferably used for a gas condensate flow.

In another embodiment, the calibration vessel is not filled separately with the different phases. Instead, the calibration vessel is filled with the multi-phase mixture from the measurement tube whereupon stratification of the multi-phase mixture takes place such that the at least one liquid phase and the at least one gaseous phase are separated in the vessel, where steps ii) and iii) are performed after stratification. This method is preferably used for a multi-phase mixture flow emanating from an oil well which usually comprises oil, water and gas.

In a preferred variant of the above described embodiment, the at least one liquid phase is separated in different kinds of liquids, particularly oil and water, during stratification, where calibration data is acquired for each kind of liquid.

In another embodiment of the invention, the multi-phase mixture is a gas condensate, where the ratio of stable and unstable condensates during atmospheric pressure is determined by the data acquisition means.

In the following, embodiments of the invention will be described in detail with respect to the accompanying drawings, wherein:
- Fig. 1: shows a front view of a multi-phase flow meter according to an embodiment of the invention;
- Fig. 2: shows a top view of the flow meter of Fig. 1;
- Fig. 3: shows a view from the left on the flow meter according to Fig. 1; and
- Fig. 4: shows a view from the right on the flow meter of Fig. 1.

The embodiment described in the following refers to a flow meter which is used at oil wells or gas wells in order to determine the composition of the multi-phase mixture flow emanating from the well. Furthermore, the flow meter can be installed in oil pipelines or gas pipelines in order to analyse the flow in those pipelines. Beside the determination of the composition of the multi-phase flow, the flow meter as described in the following also enables the determination of the flow velocity based on the method as disclosed in document WO 2011/005133 A1. However, this is an optional feature and the flow meter may only include the function of measuring the composition of the multi-phase mixture flow.

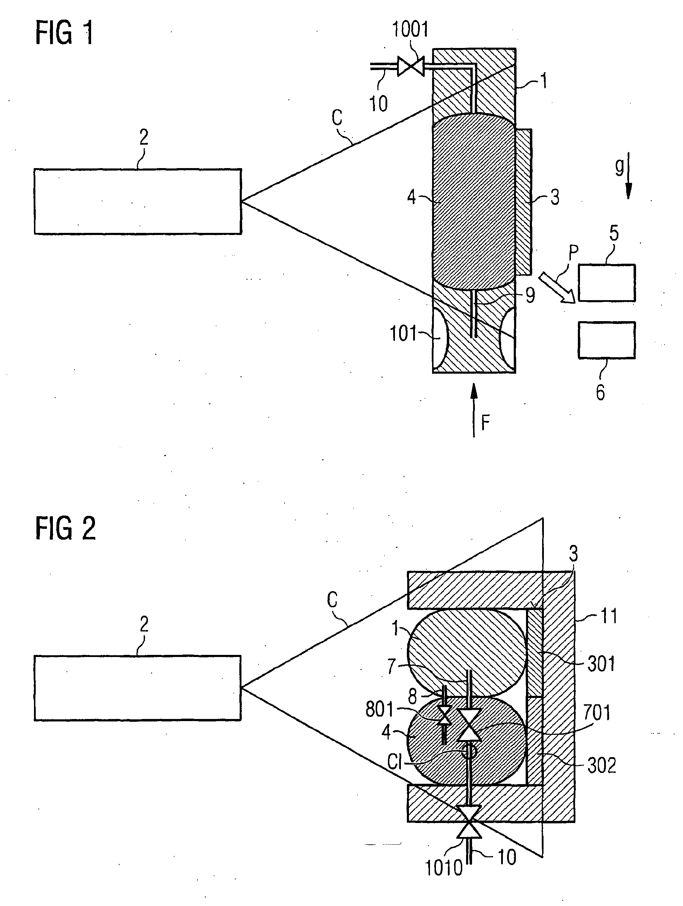

Fig. 1 shows a front view of the flow meter. The meter comprises a measurement tube 1 which is arranged in a vertical direction with respect to the force of gravity. The force of gravity is indicated by arrow g. In the measurement tube 1, a multi-phase flow is conveyed in the vertical direction upwards. The direction of this flow is indicated by arrow F. In order to analyze the phases included in the multi-phase flow, a radiation means 2 in the form of an X-ray source irradiates the measurement tube 1 with an X-ray beam. The cross-section of this beam is indicated by triangle C. The material of the measurement tube 1 is transparent for X-ray beams. Preferably, the tube is made of beryllium bronze or carbon fiber or glassy carbon or any other material being relatively transparent for X-ray radiation. A part of the radiation is absorbed by the multi-phase mixture inside the measurement tube. The rest of the radiation (i.e. the non absorbed radiation) is detected by a detection means 3 behind the measurement tube 1. The detection means comprises matrix sensors enabling a spatial resolution of the detected intensity in the vertical and the horizontal direction.

In the embodiment as shown in Fig. 1, the measurement tube is irradiated with radiation on a high energy level and a low energy level. The photons on the high energy level have an absorption coefficient which is the same for both oil and water which are included in the multi-phase flow of the well. Contrary to that, the photons on the a low energy level are absorbed significantly stronger by water than by oil. With respect to the gases in the multi-phase mixture, the absorption coefficients of all gases are very low for both levels of energy. The detection means 3 can resolve the beams with the different energy levels. By using the above described dependency between the energy levels of the photons and the absorption coefficients of the photons in the multi-phase flow, it is possible to determine the composition of the multi-phase flow. I.e., the percentages of the different materials in the flow can be determined. To do so, a common technique is used, particularly the technique described in document US 6,265,713 B1. As this technique is already known, it will not be described in the following.

For calculating the composition of the multi-phase mixture flow, the absorption coefficients for the gaseous phase and the liquid phase, preferably separately for the oil phase and the water phase and the gas phase(s), need to be known. Thus, those coefficients are calibration parameters which have to be determined in advance in a calibration process. Conventionally, the flow meter is calibrated manually. I.e., a sample is taken from the multi-phase mixture flow and analyzed in a separate process. This manual calibration is time-consuming and also requires that a person has to visit the well or the pipeline in order to take a sample. Contrary to that, the flow meter in Fig. 1 enables an automatic calibration, e.g. in regular intervals, and thus eliminates the above described disadvantages of a manual calibration.

To perform automatic calibration, the flow meter as shown in Fig. 1 includes a calibration vessel 4 which is arranged adjacent to the measurement tube 1. In the view of Fig. 1, the calibration vessel 4 is arranged in front of the measurement tube 1. The calibration vessel 4 is also irradiated by the beam C of the X-ray source 2. Furthermore, the detection means 3 also extends behind the calibration vessel 4 so that the intensity of photons passing through the calibration vessel 4 can also be detected by the detection means 3. As will be described in more detail in the following, the calibration vessel 4 is connected by a valve system including valves and conduits with the measurement tube 1. Particularly, the calibration vessel 4 can be filled with multi-phase mixture from the measurement tube 1 by opening corresponding valves which are not visible in the view of Fig. 1.

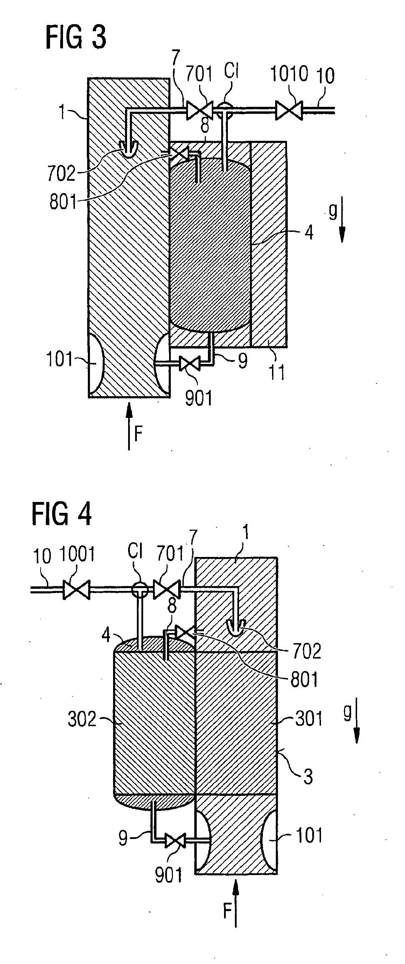

However, the view of Fig. 1 shows an upper conduit 10 with a valve 1001. This conduit is connected to the upper side of the calibration vessel 4. By opening the valve 1001, gases emanating from a multi-phase mixture inside the calibration vessel 4 can exit to the outside atmosphere. Furthermore, a lower conduit 9 is connected with the bottom side of the calibration vessel 4. This conduit includes a valve 901 (Fig. 3) which is not apparent from Fig. 1. By opening this valve, multi-phase mixture flow can be fed back from the calibration vessel 4 to the measurement tube 1. The measurement tube 1 includes in the lower part a flow restriction in the form of a ring-shaped recess 101 extending around the circumference of the tube 1. The conduit 9 is connected to the measurement tube at this flow restriction 101. Due to the Venturi effect, the pressure at the flow restriction 101 is reduced with the consequence that multi-phase mixture in the calibration vessel 4 will be blown down into the measurement tube 1.

For calibrating the flow meter, the calibration vessel 4 is filled via the valve system with multi-phase mixture from the measurement tube 1 whereupon gravitational stratification takes place thus separating the different phases and the mixture. Alternatively, the calibration vessel is separately filled with the liquid phases and gaseous phases from the multi-phase flow as will be described in more detail in the following. Afterwards, the calibration vessel is irradiated by the X-ray source 2 with the two levels of energy and the corresponding intensity of the photons passing through the calibration vessel 4 is measured by the detection means 3. As the detection means is a matrix detector having a spatial, resolution, the different phases can be distinguished due to the different absorption behavior. Eventually, the absorption coefficients for the different phases are determined and these coefficients are used as calibration data for the measurements of the multi-phase mixture flow in the measurement tube.

As indicated by arrow P in Fig. 1, the data from the detection means 3 are processed in an analysis means 5 and a data acquisition means 6 which are omitted in the other figures (though present). The analysis means performs the calculations in order to determine the composition of the multi-phase mixture based on the detected intensities of the radiation passing through the tube 1. As mentioned above, this determination is preferably based on the method as disclosed in document US 6,097,786. For the calculations, the analysis means 5 uses calibration data which are determined by data acquisition means 6. The data acquisition means obtains the detected photon intensities with respect to the radiation passing through the calibration vessel and calculates with this data in a known and straightforward manner the absorption coefficients of the different phases which are then used by the analysis means 5. The analysis means 5 and the data acquisition means 6 are implemented in hardware and software, e.g. in the form of a computer. Hence, the analysis means and the data acquisition means can form software programs installed on the same computer so that those means are integrated in one single unit.

Fig. 2 shows a top view from above on the flow meter as shown in Fig. 1. As can be seen from this Figure, the detection means 3 includes two detection sensors 301 und 302, both of which are matrix detectors. The detector 301 is for detecting radiation passing through measurement tube 1, whereas the detector 302 is for detecting radiation passing through the calibration vessel 4. In order to ensure an easy calibration, the sensitivities of both detectors are the same and the positions of detector 301 with respect to the measurement tube 1 corresponds to the position of detector 302 with respect to the calibration vessel 4. Furthermore, the cross-section and the size of the measurement tube 1 and the calibration vessel 4 are substantially the same. Moreover, the measurement tube and the vessel are formed of the same material so that the transmission for the radiation is the same for the tube and the vessel.

The choice of the cross-sectional shape of the measurement tube is based on the criteria that the tube shall withstand high pressure (optimum: circular cross-section) and that the path for different X-ray beams passing through the measurement tube to the detection means should vary as little as possible (optimum: square cross-section). Based on these criteria, an elliptic-like cross-section shape is chosen for the measurement tube 1. More precisely, the shape of the measurement tube 1 has the form of an elongated hole comprising two flat sections and two circular sections. As can be seen from Fig. 1, the calibration vessel 4 has the same form as the measurement tube 1 and is arranged adjacent to the measurement tube such that two flat surfaces of the tube and the vessel are in direct contact with each other.

As mentioned above, it is preferable that both the measurement tube 1 and the calibration vessel 4 are made of the same or similar material and have identical forms. Furthermore, the temperature of the calibration vessel 4 should be as close as possible to temperature of the measurement tube 1. To fulfill this requirement, a good thermal contact is provided via the contacting flat surfaces of the tube and the vessel. Furthermore, in order to insulate the tube and the vessel from the environment, a thermal insulation 11 is arranged around the tube and the vessel. This thermal solution was not shown in Fig. 1.

Beside the conduit 10 and the valve 1010, further conduits and valves are apparent from Fig. 2. Particularly, a conduit 7 extends inside the measurement tube 1 to a sampling probe 702 (Fig. 3). The conduit 7 includes a valve 701 and, by opening this valve, multi-phase mixture can flow via the conduit 7 to a junction indicated by circle CI. At this junction, the conduit extends on the one side down to the vessel 4 so that multi-phase mixture can enter the vessel. Furthermore, the junction extends to the conduit 10 via valve 1010. By opening this valve 1010, gases of the multi-phase mixture can exit to the outside.

Furthermore, another conduit 8 including valve 801 can be seen from Fig. 2. This conduit extends in a vertical direction in the measurement tube 1 and in a horizontal direction in the calibration vessel 4 (see also Fig. 3). By opening valve 801, the gaseous phases of the multi-phase mixture in measurement tube 1 can enter the calibration vessel 4.

Fig. 3 shows a side view from the left on the flow meter of Fig. 1. It is apparent from this Figure that the conduit 7 extends inside the measurement tube 1 and ends at a sampling probe 702. Furthermore, the junction CI between the conduit 10 and the conduit 7 can be seen. The sampling probe 702 enables multi-phase mixture inside the measurement tube to be transported to the calibration vessel 4 when valve 701 is opened. Furthermore, one can see from Fig. 3 the arrangement of the conduit 8 with corresponding valve 801 between the measurement tube 1 and the calibration vessel 4. Fig. 3 also reveals that the conduit 9 includes a valve 901 and ends in the flow restriction 101 as described above. Fig. 4 shows a view from the right on the flow meter of Fig. 1. The structure of the detection means 3 is apparent from this Figure. Particularly, one can see that the detection means includes two identical matrix detectors 301 and 302, where matrix detector 301 is arranged adjacent to the measurement tube 1 and the matrix detector 302 as arranged adjacent to a calibration vessel 4.

In the embodiment of the flow meter as described in the foregoing, the arrangement of the measurement tube 1 with respect to the X-ray source 2 and the detector 301 corresponds to the arrangement of the calibration vessel 4 with respect to the X-ray source 2 and the detector 302. Furthermore, the material and the size of the vessel and the tube are the same and the same type of detectors 301 and 302 are used. Moreover, it is assured that the multi-phase mixtures in the vessel and the tube are in the same thermal condition. As a consequence, the absorption coefficients calculated by the data acquisition means 6 can be used directly by the analysis means 5 without any conversion calculations. Hence, a very easy calibration of the flow meter is achieved, and the calibration data quality is improved.

In the following, two operation modes of the flow meter as described with respect to Fig. 1 to Fig. 4 are explained. In a both operation modes, the valves of the valve system are opened and closed in a predetermined manner. The control of the valves is performed by the data acquisition means 6 shown in Fig. 1.

In a first operation mode, the calibration of a gas condensate flow emanating from a gas well is described. In such a flow, 90 % to 95 % by volume of the mixture is gas. In a first step, the calibration of the gaseous phase is performed. To do so, valve 801 of the flow meter is opened, while all other valves are closed. As a consequence, the calibration vessel is filled with gas mixture. In this condition, the calibration data for the pure gas phase can be acquired, using the X-ray source 2 and the detection means 3.

In a next step, the liquid phase of the gas condensate is collected. To do so, valves 801 and 701 are opened while all other valves are closed. Hence, the calibration vessel 4 is filled with the liquid phase. This process can take some time because the fraction of liquid in the gas condensate is rather low in comparison to other multi-phase mixtures. After the collection of the liquid phase, calibration data for this phase are acquired via the X-ray detector 2 and the detection means 3.

In an optional step, an additional measurement can be conducted during calibration. For this additional measurement, valve 6 is opened while all other valves are closed. As a consequence, atmospheric pressure will settle down in the calibration vessel with the consequence that non-stable condensate will evaporate while stable fractions will stay in the calibration vessel. Via the measurement of the liquid level in the calibration vessel (which is possible due to the spatial resolution ability of the matrix detector 302), one can determine the ratio between stable and unstable condensates by comparing the levels in the vessel before and after opening the valve 1010.

In a final step, purging of the calibration vessel takes place. During this step, all valves except valves 801 and 901 are closed. Since the pressure in the restricted flow area 101 is reduced, the content of the calibration vessel will be blown down into the measurement tube. Thus, the calibration vessel is again filled with the gas fraction of the multi-phase mixture from the measurement tube and the above steps can be repeated again.

In a second operational mode, the calibration procedure is performed for a multi-phase flow emanating from an oil well. Such a multi-phase flow contains the phases water, gas and oil. In a first step of this mode, the valves 701 and 901 are opened. As a consequence, the multi-phase mixture will flow through the calibration vessel. The duration of this step is chosen such that the multi-phase mixture in the calibration vessel 4 will be completely exchanged by the mixture from the measurement tube. Since the calibration vessel is being filled from the top while the valve 901 is located at the bottom, the gas content of the mixture will be higher in the calibration vessel in comparison to the actual flow in the measurement tube.

In a second step, stratification of the mixture in the calibration vessel takes place. During this step, only valve 801 is opened. Due to gravitational stratification, segregation of the mixture takes place because of the different densities of oil, water and gas. The time for this step should be long enough so that the mixture is completed segregated. As a result, the calibration vessel content is distributed such that the bottom contains water followed by oil and thereafter by gas at the top.

In a next step, the data acquisition takes place with the X-ray source 2 and the detection means 3. Since oil, water and gas have different X-ray absorptions, the different phases can be distinguished by the matrix detector 302. Hence, calibration data in the form of absorption coefficients can be obtained for the three phases oil, water and gas.

The invention as described in the foregoing has several advantages. Particularly, an automatic calibration of a flow meter can be achieved, without the need to take a sample of the multi-phase mixture flow manually. The automatic calibration can be repeated in regular intervals, thus providing high quality calibration data and a very exact measurement of the composition of the multi-phase flow passing through the flow meter.

## Claims

1. An apparatus for measuring the composition of a multi-phase mixture flow comprising at least one liquid phase and/or at least one gaseous phase, particularly a flow of liquid and gaseous hydrocarbons emanating from a well, comprising:
- a measurement tube (1) forming a conduit for a flow of multi-phase mixture;
- radiation means (2) for irradiating the multi-phase mixture in the measurement tube (1) with electromagnetic radiation;
- detection means (3) for detecting the radiation of the radiation means (2) which passes through the multi-phase mixture in the measurement tube (1);
- analysis means (5) for determining the composition of the multi-phase mixture based on the detected radiation and calibration data of the at least one liquid phase and the at least one gaseous phase;
**characterised in that**
- a calibration vessel (4) is arranged adjacent to the measurement tube (1) such that the radiation means (2) can irradiate the calibration vessel (4) and the detection means (3) can detect radiation of the radiation means (2) passing through the calibration vessel (4);
- the calibration vessel (4) is connected to the measurement tube (1) such that the calibration vessel (4) is filled with the multi-phase mixture or respective phases of the multi-phase mixture from the measurement tube (1);
- data acquisition means (6) are provided for acquiring calibration data from radiation detected by the detection means (3) which passes through the calibration vessel (4) when the calibration vessel (4) is filled with the multi-phase mixture or respective phases of the multi-phase mixture from the measurement tube (1).

2. The apparatus according to claim 1, **characterised in that** the radiation of the radiation means (2) is a high energy electromagnetic radiation with a photon energy of at least 10 KeV, particularly an X-ray radiation and/or a gamma radiation.

3. The apparatus according to claim 1 or 2, **characterised in that**
- the radiation means (2) can generate at least two different radiation pulses, one pulse having a low energy level and the other pulse having a high energy level;
- the detecting means (3) can detect the different radiation pulses;
- the analysis means (5) is designed for determining the composition of the multi-phase mixture flow based on the detected different radiation pulses and the calibration data, where the calibration data is acquired by the data acquisition means (6) from the different radiation pulses detected by the detection means (3) which pass through the calibration vessel (4) when the calibration vessel (4) is filled with the multi-phase mixture or respective phases of the multi-phase mixture from the measurement tube (1).

4. The apparatus according to one of the preceding claims, **characterised in that** the calibration data comprises absorption coefficients for the phases in the multi-phase mixture with respect to the radiation of the radiation means (2).

5. The apparatus according to one of the preceding claims, **characterised in that** the measurement tube (1) and the calibration vessel (4) are made of the same material and/or have the same cross-section.

6. The apparatus according to one of the preceding claims, **characterised in that** the measurement tube (1) and/or the calibration vessel (4) are made of beryllium bronze and/or carbon fiber and/or glassy carbon.

7. The apparatus according to one of the preceding claims, **characterised in that** the measurement tube (1) and/or the calibration vessel (4) have an elliptic cross-section and/or a cross-section in the form of an elongated hole.

8. The apparatus according to one of the preceding claims, **characterised in that** the measurement tube (1) and the calibration vessel (4) are arranged symmetrically with respect to the radiation means (2) and the detection means (3) such that radiation reaching the detection means (3) which has respectively passed through the measurement tube (1) and the calibration vessel (4) does not change when the positions of the measurement tube (1) and the calibration vessel (4) are interchanged.

9. The apparatus according to one of the preceding claims, **characterised in that** a section of the measurement tube (1) and a section of the calibration vessel (4) are in direct contact with each other.

10. The apparatus according to one of the preceding claims, **characterised in that** the measurement tube (1) and the calibration vessel (4) are surrounded by a thermal insulation (11).

11. The apparatus according to one of the preceding claims, **characterised in that** the detection means (3) comprises a matrix detection means enabling a spatial resolution of the detected radiation and/or the detection means (3) comprises two detection sensors (301, 302), where one detection sensor (301) is for detecting radiation passing through the measurement tube (1) and the other detection sensor (302) is for detecting radiation passing through the calibration vessel (4).

12. The apparatus according to one of the preceding claims, **characterised in that** the apparatus is designed such that during operation of the apparatus the measurement tube (1) and the calibration vessel (4) extend in the vertical direction.

13. The apparatus according to one of the preceding claims, **characterised in that** a valve system comprising one or more valves (701, 801, 901, 1001) and one or more conduits (7, 8, 9, 10) is arranged between the measurement tube (1) and the calibration vessel (4), the valve system being controllable by the data acquisition means (6) and enabling the calibration vessel (4) to be filled with the multi-phase mixture or respective phases of the multi-phase mixture from the measurement tube (1).

14. The apparatus according to claim 13, **characterised in that** the valve system comprises a sampling probe (702) positioned in the measurement tube (1) and connected via a first conduit (7) including a first valve (701) with the calibration vessel (4), where the calibration vessel (4) is filled with the multi-phase mixture from the measurement tube (1) when the first valve (701) is opened.

15. The apparatus according to claim 13 or 14, **characterised in that** the valve system comprises a second conduit (8) including a second valve (801), where the at least one gaseous phase of the multi-phase mixture can be interchanged between the measurement tube (1) and the calibration vessel (4) when the second valve (801) is opened.

16. The apparatus according to one of claims 13 to 15, **characterised in that** the valve system comprises a third conduit (9) including a third valve (901), where multi-phase mixture in the calibration vessel (4) is fed back to the measurement tube (1) when the third valve (901) is opened, where the third conduit (9) is preferably connected to a flow restriction (101) in the measurement tube (1).

17. The apparatus according to one of claims 13 to 16, **characterised in that** the valve system comprises a fourth conduit (10) including a fourth valve (1001), where the at least one gaseous phase of the multi-phase mixture in the calibration vessel (4) is released from the calibration vessel (4) when the fourth valve (1001) is opened.

18. A method for calibrating an apparatus according to one of the preceding claims, comprising the steps of:
i) filling the calibration vessel (4) with the multi-phase mixture or respective phases of the multi-phase mixture from the measurement tube (1);
ii) irradiating the calibration vessel (4) with electromagnetic radiation from the radiation means (2) and detecting the radiation which passes through the calibration vessel (4) by the detection means (3);
iii) acquiring calibration data by the data acquisition means from the radiation detected by the detection means (3).

19. The method according to claim 18, **characterised in that** the calibration vessel (4) is subsequently filled with the at least one gaseous phase and the at least one liquid phase of the multi-phase mixture from the measurement tube (1) or vice versa, where steps ii) and iii) are performed for both the at least one liquid phase and the at least one gaseous phase or phases.

20. The method according to claim 18 or 19, **characterised in that** the calibration vessel (4) is filled with the multi-phase mixture from the measurement tube (1), whereupon stratification of the multi-phase mixture takes place such that the at least one liquid phase and the at least one gaseous phase are separated in the vessel, where steps ii) and iii) are performed after the stratification.

21. The method according to claim 20, **characterised in that** the at least one liquid phase is separated in different kinds of liquids, particularly oil and water, during stratification, where calibration data is acquired for each kind of liquid.

22. The method according to one of claims 18 to 20, **characterised in that** the multi-phase mixture is a gas condensate, where the ratio of stable and unstable condensate during atmospheric pressure is determined by the data acquisition means (6).

## Patentansprüche

1. Vorrichtung zum Messen der Zusammensetzung eines Mehrphasengemischstroms, der wenigstens eine flüssige Phase und/oder wenigstens eine gasförmige Phase umfasst, insbesondere eines aus einem Bohrloch austretenden Stroms von flüssigen und gasförmigen Kohlenwasserstoffen, welche umfasst:
- ein Messrohr (1), das einen Kanal für einen Strom eines Mehrphasengemisches bildet;
- Strahlungsmittel (2) zum Bestrahlen des Mehrphasengemisches in dem Messrohr (1) mit elektromagnetischer Strahlung;
- Detektionsmittel (3) zum Detektieren der Strahlung des Strahlungsmittels (2), welche das Mehrphasengemisch in dem Messrohr (1) durchdringt;
- Analysemittel (5) zum Bestimmen der Zusammensetzung des Mehrphasengemisches auf der Basis der detektierten Strahlung und von Kalibrierungsdaten der wenigstens einen flüssigen Phase und der wenigstens einen gasförmigen Phase;
**dadurch gekennzeichnet, dass**
- ein Kalibriergefäß (4) dem Messrohr (1) benachbart angeordnet ist, so dass die Strahlungsmittel (2) das Kalibriergefäß (4) bestrahlen können und die Detektionsmittel (3) Strahlung der Strahlungsmittel (2) detektieren können, welche das Kalibriergefäß (4) durchdringt;
- das Kalibriergefäß (4) mit dem Messrohr (1) verbunden ist, so dass das Kalibriergefäß (4) mit dem Mehrphasengemisch oder jeweiligen Phasen des Mehrphasengemisches aus dem Messrohr (1) gefüllt wird;
- Datenerfassungsmittel (6) zum Erfassen von Kalibrierdaten von durch die Detektionsmittel (3) detektierter Strahlung vorgesehen sind, welche das Kalibriergefäß (4) durchdringt, wenn das Kalibriergefäß (4) mit dem Mehrphasengemisch oder jeweiligen Phasen des Mehrphasengemisches aus dem Messrohr (1) gefüllt wird.

2. Vorrichtung nach Anspruch 1, **dadurch gekennzeichnet, dass** die Strahlung der Strahlungsmittel (2) eine hochenergetische elektromagnetische Strahlung mit einer Photonenenergie von wenigstens 10 keV ist, insbesondere eine Röntgenstrahlung und/oder Gammastrahlung.

3. Vorrichtung nach Anspruch 1 oder 2, **dadurch gekennzeichnet, dass**
- die Strahlungsmittel (2) wenigstens zwei verschiedene Strahlungsimpulse erzeugen können, wobei ein Impuls einen niedrigen Energiepegel aufweist und der andere Impuls einen hohen Energiepegel aufweist;
- die Detektionsmittel (3) die verschiedenen Strahlungsimpulse detektieren können;
- die Analysemittel (5) dafür ausgebildet sind, die Zusammensetzung des Mehrphasengemischstroms auf der Basis der detektierten verschiedenen Strahlungsimpulse und der Kalibrierdaten zu bestimmen, wobei die Kalibrierdaten durch die Datenerfassungsmittel (6) aus den von den Detektiermitteln (3) detektierten verschiedenen Strahlungsimpulsen erfasst werden, welche das Kalibriergefäß (4) durchdringen, wenn das Kalibriergefäß (4) mit dem Mehrphasengemisch oder jeweiligen Phasen des Mehrphasengemisches aus dem Messrohr (1) gefüllt wird.

4. Vorrichtung nach einem der vorhergehenden Ansprüche, **dadurch gekennzeichnet, dass** die Kalibrierdaten Absorptionskoeffizienten für die Phasen in dem Mehrphasengemisch in Bezug auf die Strahlung der Strahlungsmittel (2) umfassen.

5. Vorrichtung nach einem der vorhergehenden Ansprüche, **dadurch gekennzeichnet, dass** das Messrohr (1) und das Kalibriergefäß (4) aus demselben Material hergestellt sind und/oder denselben Querschnitt aufweisen.

6. Vorrichtung nach einem der vorhergehenden Ansprüche, **dadurch gekennzeichnet, dass** das Messrohr (1) und/oder das Kalibriergefäß (4) aus Berylliumbronze und/oder Kohlenstofffasern und/oder Glaskohlenstoff hergestellt sind.

7. Vorrichtung nach einem der vorhergehenden Ansprüche, **dadurch gekennzeichnet, dass** das Messrohr (1) und/oder das Kalibriergefäß (4) einen elliptischen Querschnitt und/oder einen Querschnitt in der Form eines lang gestreckten Loches aufweisen.

8. Vorrichtung nach einem der vorhergehenden Ansprüche, **dadurch gekennzeichnet, dass** das Messrohr (1) und das Kalibriergefäß (4) symmetrisch in Bezug auf die Strahlungsmittel (2) und die Detektionsmittel (3) angeordnet sind, so dass die die Detektionsmittel (3) erreichende Strahlung, welche das Messrohr (1) bzw. das Kalibriergefäß (4) durchdrungen hat, sich nicht ändert, wenn die Positionen des Messrohrs (1) und des Kalibriergefäßes (4) miteinander vertauscht werden.

9. Vorrichtung nach einem der vorhergehenden Ansprüche, **dadurch gekennzeichnet, dass** sich ein Abschnitt des Messrohrs (1) und ein Abschnitt des Kalibriergefäßes (4) in direktem Kontakt miteinander befinden.

10. Vorrichtung nach einem der vorhergehenden Ansprüche, **dadurch gekennzeichnet, dass** das Messrohr (1) und das Kalibriergefäß (4) von einer Wärmeisolation (11) umgeben sind.

11. Vorrichtung nach einem der vorhergehenden Ansprüche, **dadurch gekennzeichnet, dass** die Detektionsmittel (3) ein Matrix-Detektionsmittel umfassen, das eine räumliche Auflösung der detektierten Strahlung ermöglicht, und/oder die Detektionsmittel (3) zwei Detektionssensoren (301, 302) umfassen, wobei ein Detektionssensor (301) zum Detektieren von Strahlung bestimmt ist, welche das Messrohr (1) durchdringt, und der andere Detektionssensor (302) zum Detektieren von Strahlung bestimmt ist, welche das Kalibriergefäß (4) durchdringt.

12. Vorrichtung nach einem der vorhergehenden Ansprüche, **dadurch gekennzeichnet, dass** die Vorrichtung derart gestaltet ist, dass sich das Messrohr (1) und das Kalibriergefäß (4) während des Betriebs der Vorrichtung in der vertikalen Richtung erstrecken.

13. Vorrichtung nach einem der vorhergehenden Ansprüche, **dadurch gekennzeichnet, dass** ein Ventilsystem, welches ein oder mehrere Ventile (701, 801, 901, 1001) und einen oder mehrere Kanäle (7, 8, 9, 10) umfasst, zwischen dem Messrohr (1) und dem Kalibriergefäß (4) angeordnet ist, wobei das Ventilsystem durch die Datenerfassungsmittel (6) steuerbar ist und ermöglicht, dass das Kalibriergefäß (4) mit dem Mehrphasengemisch oder jeweiligen Phasen des Mehrphasengemisches aus dem Messrohr (1) gefüllt wird.

14. Vorrichtung nach Anspruch 13, **dadurch gekennzeichnet, dass** das Ventilsystem eine Probenahmesonde (702) umfasst, die in dem Messrohr (1) positioniert ist und über einen ersten Kanal (7), der ein erstes Ventil (701) aufweist, mit dem Kalibriergefäß (4) verbunden ist, wobei das Kalibriergefäß (4) mit dem Mehrphasengemisch aus dem Messrohr (1) gefüllt wird, wenn das erste Ventil (701) geöffnet wird.

15. Vorrichtung nach Anspruch 13 oder 14, **dadurch gekennzeichnet, dass** das Ventilsystem einen zweiten Kanal (8) umfasst, der ein zweites Ventil (801) aufweist, wobei die wenigstens eine gasförmige Phase des Mehrphasengemisches zwischen dem Messrohr (1) und dem Kalibriergefäß (4) ausgetauscht werden kann, wenn das zweite Ventil (801) geöffnet wird.

16. Vorrichtung nach einem der Ansprüche 13 bis 15, **dadurch gekennzeichnet, dass** das Ventilsystem einen dritten Kanal (9) umfasst, der ein drittes Ventil (901) aufweist, wobei Mehrphasengemisch in dem Kalibriergefäß (4) zu dem Messrohr (1) zurückgeführt wird, wenn das dritte Ventil (901) geöffnet wird, wobei der dritte Kanal (9) vorzugsweise mit einer Durchflussbegrenzung (101) in dem Messrohr (1) verbunden ist.

17. Vorrichtung nach einem der Ansprüche 13 bis 16, **dadurch gekennzeichnet, dass** das Ventilsystem einen vierten Kanal (10) umfasst, der ein viertes Ventil (1001) aufweist, wobei die wenigstens eine gasförmige Phase des Mehrphasengemisches in dem Kalibriergefäß (4) aus dem Kalibriergefäß (4) freigegeben wird, wenn das vierte Ventil (1001) geöffnet wird.

18. Verfahren zum Kalibrieren einer Vorrichtung nach einem der vorhergehenden Ansprüche, welche die folgenden Schritte umfasst:
i) Füllen des Kalibriergefäßes (4) mit dem Mehrphasengemisch oder jeweiligen Phasen des Mehrphasengemisches aus dem Messrohr (1);
ii) Bestrahlen des Kalibriergefäßes (4) mit elektromagnetischer Strahlung von den Strahlungsmitteln (2) und Detektieren der Strahlung, welche das Kalibriergefäß (4) durchdringt, durch die Detektionsmittel (3) ;
iii) Erfassen von Kalibrierdaten von der durch die Detektionsmittel (3) detektierten Strahlung durch die Datenerfassungsmittel.

19. Verfahren nach Anspruch 18, **dadurch gekennzeichnet, dass** das Kalibriergefäß (4) nacheinander mit der wenigstens einen gasförmigen Phase und der wenigstens einen flüssigen Phase des Mehrphasengemisches aus dem Messrohr (1) gefüllt wird, oder umgekehrt, wobei die Schritte ii) und iii) für sowohl die wenigstens eine flüssige Phase als auch die wenigstens eine gasförmige Phase oder Phasen ausgeführt werden.

20. Verfahren nach Anspruch 18 oder 19, **dadurch gekennzeichnet, dass** das Kalibriergefäß (4) mit dem Mehrphasengemisch aus dem Messrohr (1) gefüllt wird, woraufhin eine Schichtenbildung des Mehrphasengemisches stattfindet, so dass die wenigstens eine flüssige Phase und die wenigstens eine gasförmige Phase in dem Gefäß getrennt werden, wobei die Schritte ii) und iii) nach der Schichtenbildung ausgeführt werden.

21. Verfahren nach Anspruch 20, **dadurch gekennzeichnet, dass** die wenigstens eine flüssige Phase während der Schichtenbildung in verschiedene Arten von Flüssigkeiten getrennt wird, insbesondere Öl und Wasser, wobei Kalibrierdaten für jede Flüssigkeitsart erfasst werden.

22. Verfahren nach einem der Ansprüche 18 bis 20, **dadurch gekennzeichnet, dass** das Mehrphasengemisch ein Gaskondensat ist, wobei das Verhältnis von stabilem und instabilem Kondensat bei atmosphärischem Druck durch die Datenerfassungsmittel (6) bestimmt wird.

## Revendications

1. Appareil permettant de mesurer la composition d'un flux de mélange multiphasé comprenant au moins une phase liquide et/ou au moins une phase gazeuse, en particulier un flux d'hydrocarbures liquides et gazeux émanant d'un puits, comprenant :
- un tube de mesure (1) formant un conduit pour un flux de mélange multiphasé ;
- un moyen de rayonnement (2) permettant d'irradier le mélange multiphasé dans le tube de mesure (1) avec un rayonnement électromagnétique ;
- un moyen de détection (3) permettant de détecter le rayonnement du moyen de rayonnement (2) qui passe à travers le mélange multiphasé dans le tube de mesure (1) ;
- un moyen d'analyse (5) permettant de déterminer la composition du mélange multiphasé sur la base du rayonnement détecté et des données d'étalonnage de l'au moins une phase liquide et de l'au moins une phase gazeuse ;
**caractérisé en ce que**
- un récipient d'étalonnage (4) est disposé de manière adjacente au tube de mesure (1) de telle sorte que le moyen de rayonnement (2) peut irradier le récipient d'étalonnage (4) et le moyen de détection (3) peut détecter un rayonnement du moyen de rayonnement (2) passant à travers le récipient d'étalonnage (4) ;
- le récipient d'étalonnage (4) est raccordé au tube de mesure (1) de telle sorte que le récipient d'étalonnage (4) est rempli avec le mélange multiphasé ou les phases respectives du mélange multiphasé du tube de mesure (1) ;
- des moyens d'acquisition de données (6) sont prévus pour acquérir des données d'étalonnage provenant du rayonnement détecté par le moyen de détection (3) qui passe à travers le récipient d'étalonnage (4) lorsque le récipient d'étalonnage (4) est rempli avec le mélange multiphasé ou les phases respectives du mélange multiphasé du tube de mesure (1).

2. Appareil selon la revendication 1, **caractérisé en ce que** le rayonnement du moyen de rayonnement (2) est un rayonnement électromagnétique à forte énergie avec une énergie de photons d'au moins 10 KeV, en particulier un rayonnement par rayons X et/ou un rayonnement gamma.

3. Appareil selon la revendication 1 ou 2, **caractérisé en ce que**
- le moyen de rayonnement (2) peut générer au moins deux impulsions de rayonnement différentes, une impulsion ayant un niveau d'énergie faible et l'autre impulsion ayant un niveau d'énergie élevé ;
- le moyen de détection (3) peut détecter les différentes impulsions de rayonnement ;
- le moyen d'analyse (5) est conçu pour déterminer la composition du flux de mélange multiphasé sur la base des différentes impulsions de rayonnement détectés et des données d'étalonnage, où les données d'étalonnage sont acquises par le moyen d'acquisition de données (6) à partir des différentes impulsions de rayonnement détectées par le moyen de détection (3) qui passent à travers le récipient d'étalonnage (4) lorsque le récipient d'étalonnage (4) est rempli avec le mélange multiphasé ou les phases respectives du mélange multiphasé du tube de mesure (1).

4. Appareil selon l'une des revendications précédentes, **caractérisé en ce que** les données d'étalonnage comprennent des coefficients d'absorption pour les phases dans le mélange multiphasé par rapport au rayonnement du moyen de rayonnement (2).

5. Appareil selon l'une des revendications précédentes, **caractérisé en ce que** le tube de mesure (1) et le récipient d'étalonnage (4) sont constitués du même matériau et/ou ont la même coupe transversale.

6. Appareil selon l'une des revendications précédentes, **caractérisé en ce que** le tube de mesure (1) et/ou le récipient d'étalonnage (4) sont constitués de bronze au béryllium et/ou de fibre de carbone et/ou de carbone vitreux.

7. Appareil selon l'une des revendications précédentes, **caractérisé en ce que** le tube de mesure (1) et/ou le récipient d'étalonnage (4) ont une coupe transversale elliptique et/ou une coupe transversale ayant la forme d'un trou allongé.

8. Appareil selon l'une des revendications précédentes, **caractérisé en ce que** le tube de mesure (1) et le récipient d'étalonnage (4) sont disposés de manière symétrique par rapport au moyen de rayonnement (2) et au moyen de détection (3) de telle sorte que le rayonnement atteignant le moyen de détection (3) qui est passé respectivement à travers le tube de mesure (1) et le récipient d'étalonnage (4) ne change pas lorsque les positions du tube de mesure (1) et du récipient d'étalonnage (4) sont échangées.

9. Appareil selon l'une des revendications précédentes, **caractérisé en ce qu'**une section du tube de mesure (1) et une section du récipient d'étalonnage (4) sont en contact direct l'une avec l'autre.

10. Appareil selon l'une des revendications précédentes, **caractérisé en ce que** le tube de mesure (1) et le récipient d'étalonnage (4) sont entourés par une isolation thermique (11).

11. Appareil selon l'une des revendications précédentes, **caractérisé en ce que** le moyen de détection (3) comprend un moyen de détection de matrice permettant une résolution spatiale du rayonnement détecté et/ou le moyen de détection (3) comprend deux capteurs de détection (301, 302), lorsqu'un capteur de détection (301) est destiné à détecter un rayonnement passant à travers le tube de mesure (1) et l'autre capteur de détection (302) est destiné à détecter un rayonnement passant à travers le récipient d'étalonnage (4).

12. Appareil selon l'une des revendications précédentes, **caractérisé en ce que** l'appareil est conçu de telle sorte que pendant le fonctionnement de l'appareil le tube de mesure (1) et le récipient d'étalonnage (4) s'étendent dans la direction verticale.

13. Appareil selon l'une des revendications précédentes, **caractérisé en ce qu'**un système de soupapes comprenant une ou plusieurs soupapes (701, 801, 901, 1001) et un ou plusieurs conduits (7, 8, 9, 10) sont disposés entre le tube de mesure (1) et le récipient d'étalonnage (4), le système de soupapes pouvant être commandé par le moyen d'acquisition de données (6) et permettant de remplir le récipient d'étalonnage (4) avec le mélange multiphasé ou les phases respectives du mélange multiphasé du tube de mesure (1).

14. Appareil selon la revendication 13, **caractérisé en ce que** le système de soupapes comprend une sonde d'échantillonnage (702) positionnée dans le tube de mesure (1) et raccordée via un premier conduit (7) comprenant une première soupape (701) avec le récipient d'échantillonnage (4), où le récipient d'étalonnage (4) est rempli avec le mélange multiphasé du tube de mesure (1) lorsque la première soupape (701) est ouverte.

15. Appareil selon la revendication 13 ou 14, **caractérisé en ce que** le système de soupapes comprend un deuxième conduit (8) comprenant une deuxième soupape (801), où l'au moins une phase gazeuse du mélange multiphasé peut être échangée entre le tube de mesure (1) et le récipient d'étalonnage (4) lorsque la deuxième soupape (801) est ouverte.

16. Appareil selon l'une des revendications 13 à 15, **caractérisé en ce que** le système de soupapes comprend un troisième conduit (9) comprenant une troisième soupape (901), où le mélange multiphasé dans le récipient d'étalonnage (4) est ramené dans le tube de mesure (1) lorsque la troisième soupape (901) est ouverte, où le troisième conduit (9) est de préférence raccordé à une réduction de flux (101) dans le tube de mesure (1).

17. Appareil selon l'une des revendications 13 à 16, **caractérisée en ce que** le système de soupapes comprend un quatrième conduit (10) comprenant une quatrième soupape (1001), où l'au moins une phase gazeuse du mélange multiphasé dans le récipient d'étalonnage (4) est libérée du récipient d'étalonnage (4) lorsque la quatrième soupape (1001) est ouverte.

18. Procédé permettant d'étalonner un appareil selon l'une des revendications précédentes, comprenant les étapes consistant à :
i) remplir le récipient d'étalonnage (4) avec le mélange multiphasé ou les phases respectives du mélange multiphasé du tube de mesure (1) ;
ii) irradier le récipient d'étalonnage (4) avec un rayonnement électromagnétique provenant du moyen de rayonnement (2) et détecter le rayonnement qui passe à travers le récipient d'étalonnage (4) par le moyen de détection (3) ;
iii) acquérir des données d'étalonnage par le moyen d'acquisition de données dans le rayonnement détecté par le moyen de détection (3).

19. Procédé selon la revendication 18, **caractérisé en ce que** le récipient d'étalonnage (4) est par la suite rempli avec l'au moins une phase gazeuse et l'au moins une phase liquide du mélange multiphasé du tube de mesure (1) ou inversement, où les étapes ii) et iii) sont exécutées pour les deux parmi l'au moins une phase liquide et l'au moins une phase gazeuse ou les phases.

20. Procédé selon la revendication 18 ou 19, **caractérisé en ce que** le récipient d'étalonnage (4) est rempli avec le mélange multiphasé du tube de mesure (1), après quoi une stratification du mélange multiphasé se produit de telle sorte que l'au moins une phase liquide et l'au moins une phase gazeuse sont séparées dans le récipient, où les étapes ii) et iii) sont réalisées après la stratification.

21. Procédé selon la revendication 20, **caractérisé en ce que** l'au moins une phase liquide est séparée en différents types de liquides, en particulier de l'huile et de l'eau, pendant la stratification, où des données d'étalonnage sont acquises pour chaque type de liquide.

22. Procédé selon l'une des revendications 18 à 20, **caractérisé en ce que** le mélange multiphasé est un condensat de gaz, où le rapport entre condensat stable et instable sous pression atmosphérique est déterminé par le moyen d'acquisition de données (6).
